# FASCICULE DE BREVET EUROPEEN

(11) **EP 1 353 891 B1**
(45) Date de publication et mention de la délivrance du brevet: **05.10.2016**
(21) Numéro de dépôt: 02710951.1
(22) Date de dépôt: 08.01.2002
(51) Int. Cl.: C07C 39/16, C07C 37/20

(54) **PROCÉDÉ DE FABRICATION DU BISPHÉNOL A**
VERFAHREN ZUR HERSTELLUNG VON BISPHENOL A
METHOD FOR MAKING BISPHENOL A

(30) Priorité: 23.01.2001 FR 0100882
(43) Date de publication de la demande: 22.10.2003
(73) Titulaire: ARKEMA FRANCE, 92700 Colombes (FR)
(72) Inventeur: COMMARIEU, Annie, F-64000 Pau (FR)
(74) Mandataire: Lhoste, Catherine
(86) Numéro de dépôt international: PCT/FR2002/000042
(87) Numéro de publication internationale: WO 2002/059069

(56) Documents cités:
- AU-B2- 474 155
- US-A- 4 517 387
- 'Mercaptals', [en ligne] 1997, IUPAC COMPENDIUM OF CHEMICAL TERMINOLOGY, ELECTRONIC VERSION Extrait de l'Internet: <URL:http://goldbook.iupac.org/M03830.pdf>
- 'Mercaptoles', [en ligne] 1997, IUPAC COMPENDIUM OF CHEMICAL TERMINOLOGY, ELECTRONIC VERSION Extrait de l'Internet: <URL:http://goldbook.iupac.org/M03833.pdf>

## Description

La présente invention concerne le domaine des bisphénols et a plus particulièrement pour objet la fabrication du bisphénol A (4,4'-isopropylidènediphénol).

Le bisphénol A est le bisphénol le plus utilisé industriellement. Produit à plus d'un million de tonnes, il est principalement employé comme intermédiaire dans la fabrication de matières plastiques, notamment celle des polycarbonates, des résines époxy et des polyesters.

Le bisphénol A est un produit connu depuis longtemps et la littérature relative à sa synthèse est abondante (voir par exemple Ullmann's Encyclopedia of Industrial Chemistry 5ème édition, vol. A 19, pp. 350-351). Industriellement, le bisphénol A est le plus souvent obtenu par condensation d'une mole d'acétone avec 2 moles de phénol selon la réaction:

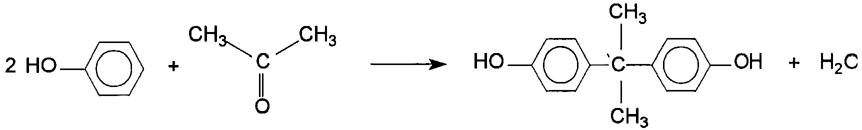

Cette réaction est généralement effectuée en présence d'un catalyseur acide fort (HCl ou une résine sulfonique) et d'un co-catalyseur mercaptan tel que le méthylmercaptan (MM) ou l'acide 3-mercaptopropionique. En l'absence de catalyseur mercaptan, la réaction est possible, mais elle est plus lente et moins sélective, l'isomère 2,4' se formant alors plus facilement.

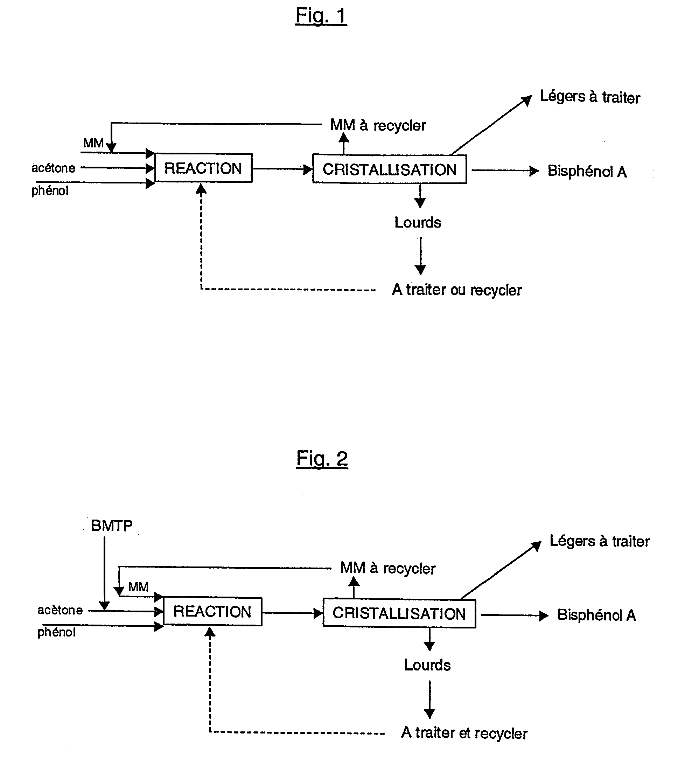

La figure 1 annexée représente un schéma de principe d'un procédé industriel classique de fabrication du bisphénol A. Ce procédé comprend essentiellement une zone réactionnelle alimentée en acétone, phénol, acide fort et mercaptan (frais et recyclé) et une zone de cristallisation du bisphénol A avec des moyens pour la séparation des légers et des lourds et pour le recyclage du mercaptan (MM).

Dans un tel procédé, la totalité du mercaptan ne peut pas être recyclée et il est nécessaire d'introduire un appoint de mercaptan frais, appoint qui peut atteindre 99 % de la quantité totale nécessaire.

Malheureusement, en raison de sa forte toxicité, les livraisons de méthylmercaptan posent de tels problèmes de sécurité que les fabricants de bisphénol A qui utilisent le méthylmercaptan comme co-catalyseur sont à la recherche de solutions de remplacement du méthylmercaptan. Il a ainsi été proposé dans le brevet US 4 517 387 de remplacer l'alimentation en méthylmercaptan par une injection directe de méthylmercaptide de sodium (SMM) dans la réaction de synthèse du bisphénol A. Ce mode opératoire paraît difficilement utilisable à l'échelle industrielle car il peut générer un dépôt de sels de sodium dans le réacteur. Une autre solution envisagée consisterait à générer, dans une installation spécifique du site utilisateur, le méthylmercaptan à partir de SMM avant de l'injecter dans le réacteur de synthèse du bisphénol A. La mise en oeuvre de cette solution est cependant relativement lourde puisqu'elle nécessite l'installation d'un réacteur supplémentaire pour neutraliser le SMM par un acide fort et génère des effluents aqueux salins malodorants.

Il a maintenant été trouvé qu'on peut résoudre le problème en remplaçant le méthylmercaptan par un dithioéther de formule générale (I), ce produit étant obtenu par condensation d'une molécule d'un dérivé carbonylé (II) avec deux molécules d'un mercaptan (III) :

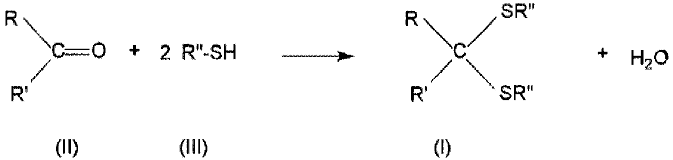

Synthétisés ex situ sur le site de production du mercaptan, les dithioéthers sont des produits faciles à manipuler. Liquides aux températures habituellement 15 utilisées pour la synthèse du bisphénol A (25-180°C), les dithioéthers liquides peuvent être introduits directement dans le réacteur de synthèse du bisphénol A au moyen d'une pompe doseuse ; leur emploi ne nécessite donc aucune modification substantielle de l'installation existante de fabrication du bisphénol A. De plus, au contraire du SMM, les dithioéthers présentent l'avantage de ne pas générer d'effluents alcalins malodorants.

L'invention a donc pour objet un procédé continu de fabrication du bisphénol A par réaction du phénol et de l'acétone en présence d'un catalyseur acide fort et d'un co-catalyseur, caractérisé en ce que l'on utilise comme co-catalyseur un dithioéther qui est le 2,2-bis(méthylthio)propane (encore appelé 3,3-diméthyl-2,4-dithiapentane et ci-après désigné par l'abréviation BMTP) qui est le produit de condensation d'une molécule d'acétone avec deux molécules de méthylmercaptan :

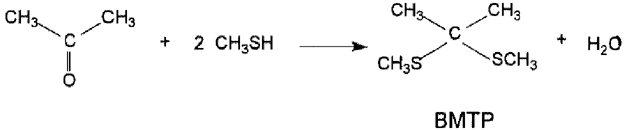

Ce dithioéther présente les caractéristiques suivantes :

| | BMTP |
|---|---|
| Température de cristallisation | - 18°C |
| Température d'ébullition | 155°C |

On peut considérer que le BMTP est un équivalent du méthylmercaptan. On peut donc l'utiliser dans une installation existante de fabrication du bisphénol A dans les mêmes conditions que le méthylmercaptan, en travaillant à isoconcentration de soufre dans le mélange réactionnel. Sachant qu'une mole de thioéther correspond à 2 moles de méthylmercaptan, 1 kg de BMTP équivaut, en activité, à 0,7 kg de méthylmercaptan.

Étant donné que, dans un procédé industriel classique de fabrication du bisphénol A, la concentration en méthylmercaptan du mélange réactionnel est comprise entre 1 et 100 % molaire par rapport au catalyseur acide fort (résine sulfonique, acide chlorhydrique ou acide sulfurique), la quantité de dithioéther à utiliser sera comprise entre 0,5 et 50 % molaire par rapport au catalyseur acide fort, de préférence entre 2,5 et 25%.

Les dithioéthers utilisés peuvent être synthétisés par tout moyen connu, mais ils le sont avantageusement en injectant le mercaptan (III) dans le dérivé carbonylé (II) à température ambiante en présence d'un catalyseur acide fort tel que l'acide chlorhydrique, l'acide sulfurique ou un solide acide comme une résine sulfonique (par exemple la résine Amberlyst^{®}15) ou une zéolithe, puis en éliminant à l'évaporateur rotatif ou par distillation le dérivé carbonylé éventuellement en excès et l'eau produite. Le mélange réactionnel en fin de synthèse est composé essentiellement du dithioéther, d'eau et du dérivé carbonylé non réagi. Il peut aussi être injecté tel quel (c'est-à-dire le dithioéther en mélange avec l'eau et le dérivé carbonylé) dans le procédé bisphénol A (surtout quand le dithioéther est le BMTP car tous les composants du mélange sont présents dans le milieu réactionnel).

Dans le procédé selon l'invention, la synthèse du bisphénol A s'effectue exactement dans les mêmes conditions opératoires que celles utilisées quand le cocatalyseur est le méthylmercaptan, à savoir :
- rapport molaire phénol/acétone: 1 à 50, de préférence 5 à 20
- température : 25-180°C, de préférence 40 à 160°C
- pression : 0,5-20 bars, de préférence 1 à 10 bars
- temps de contact : 5-180 minutes, de préférence 10 à 120 minutes.

Etant donné qu'au cours de la réaction le BMTP génère du méthylmercaptan, on peut utiliser sans aucune modification le train de séparation habituel pour collecter les effluents gazeux, puis recycler ou neutraliser par de la soude.

Comme indiqué précédemment, le remplacement du méthylmercaptan par le BMTP ne nécessite pas de modifications substantielles de l'installation existante de fabrication du bisphénol A, la seule modification étant, comme montré sur la figure 2 annexée, une entrée de BMTP liquide au lieu d'une entrée de méthylmercaptan.

Conformément à un mode particulier de mise en oeuvre du procédé selon l'invention, on peut remplacer l'injection de BMTP pur par celle d'un mélange de BMTP, d'acétone et d'eau tel que, par exemple, celui résultant de la synthèse ex-situ du BMTP par réaction de méthylmercaptan avec un excès d'acétone. Il suffit de tenir compte de l'acétone présent dans ce mélange pour régler l'alimentation du réacteur en acétone frais. Le mélange BMTP-acétone-eau est aussi facilement transportable que le BMTP pur et on évite ainsi l'opération de purification du BMTP.

De même, l'injection de BMTM pur peut être remplacée par celle d'un mélange de BMTM, de formol et d'eau tel que celui résultant de la synthèse ex-situ du BMTM par réaction de méthylmercaptan avec un excès de formol.

## Revendications

1. Procédé continu de fabrication du bisphénol A par réaction du phénol et de l'acétone en présence d'un catalyseur acide fort et d'un co-catalyseur, **caractérisé en ce que** l'on utilise comme co-catalyseur un dithioéther qui est le 2,2-bis(méthylthio)propane.

2. Procédé selon la revendication 1, dans lequel on utilise du 2,2-bis(méthylthio)propane pur.

3. Procédé selon la revendication 1, dans lequel on utilise un mélange de 2,2-bis(méthylthio)propane, d'acétone et d'eau.

4. Procédé selon la revendication 3, dans lequel le mélange est celui résultant de la synthèse ex-situ du 2,2-bis(méthylthio)propane par réaction du méthylmercaptan avec de l'acétone.

5. Procédé selon l'une des revendications 1 à 4, dans lequel la quantité de dithioéther utilisée est comprise entre 0,5 et 50% molaire par rapport au catalyseur acide fort, de préférence entre 2,5 et 25%.

6. Procédé selon l'une des revendications 1 à 5, dans lequel on opère à une température comprise entre 25 et 180°C, de préférence entre 40 et 160°C.

7. Procédé selon l'une des revendications 1 à 6, dans lequel on travaille à une pression comprise entre 0,5 et 20 bars, de préférence entre 1 et 10 bars.

8. Procédé selon l'une des revendications 1 à 7, dans lequel le temps de contact est compris entre 5 et 180 minutes, de préférence entre 10 et 120 minutes.

## Patentansprüche

1. Kontinuierliches Verfahren zur Herstellung von Bisphenol A durch Umsetzung von Phenol und Aceton in Gegenwart eines stark sauren Katalysators und eines Cokatalysators, **dadurch gekennzeichnet, dass** man als Cokatalysator einen Dithioether verwendet, bei dem es sich um 2,2-Bis(methylthio)propan handelt.

2. Verfahren nach Anspruch 1, bei dem man reines 2,2-Bis(methylthio)propan verwendet.

3. Verfahren nach Anspruch 1, bei dem man eine Mischung von 2,2-Bis(methylthio)propan, Aceton und Wasser verwendet.

4. Verfahren nach Anspruch 3, bei dem es sich bei der Mischung um diejenige handelt, die bei der ex-situ-Synthese von 2,2-Bis(methylthio)propan durch Umsetzung von Methylmercaptan mit Aceton anfällt.

5. Verfahren nach einem der Ansprüche 1 bis 4, bei dem die verwendete Dithioether-Menge zwischen 0,5 und 50 Mol-%, bezogen auf den stark sauren Katalysator, vorzugsweise zwischen 2,5 um 25 Mol-%, liegt.

6. Verfahren nach einem der Ansprüche 1 bis 5, bei dem man bei einer Temperatur zwischen 25 und 180°C, vorzugsweise zwischen 40 und 160°C, arbeitet.

7. Verfahren nach einem der Ansprüche 1 bis 6, bei dem man bei einem Druck zwischen 0,5 und 20 bar, vorzugsweise zwischen 1 und 10 bar, arbeitet.

8. Verfahren nach einem der Ansprüche 1 bis 7, bei dem die Kontaktzeit zwischen 5 und 180 Minuten, vorzugsweise zwischen 10 und 120 Minuten, liegt.

## Claims

1. Continuous process for manufacturing bisphenol A by reacting phenol and acetone in the presence of a strong acid catalyst and a co-catalyst, **characterized in that** a dithio ether which is 2,2-bis(methylthio)propane.

2. Process according to Claim 1, in which pure 2,2-bis(methylthio)propane is used.

3. Process according to Claim 1, in which a mixture of 2,2-bis(methylthio)propane, acetone and water is used.

4. Process according to Claim 3, in which the mixture is that resulting from the ex situ synthesis of 2,2-bis(methylthio)propane by reacting methyl mercaptan with acetone.

5. Process according to one of Claims 1 to 4, in which the amount of dithio ether used is between 0.5 mol% and 50 mol% relative to the strong acid catalyst, and preferably between 2.5 mol% and 25 mol%.

6. Process according to one of Claims 1 to 5, which is performed at a temperature of between 25 and 180°C and preferably between 40 and 160°C.

7. Process according to one of Claims 1 to 6, in which the working pressure is between 0.5 and 20 bar and preferably between 1 and 10 bar.

8. Process according to one of Claims 1 to 7, in which the contact time is between 5 and 180 minutes and preferably between 10 and 120 minutes.
